Europäisches Patentamt

European Patent Office

Office européen des brevets

(19)

(11) **EP 0 683 237 B1**

(12) **EUROPEAN PATENT SPECIFICATION**

(45) Date of publication and mention
of the grant of the patent:
**06.03.2002 Bulletin 2002/10**

(51) Int Cl.[7]: **C12Q 1/25**

(86) International application number:
**PCT/JP94/00779**

(21) Application number: **94914618.7**

(22) Date of filing: **13.05.1994**

(87) International publication number:
**WO 95/15398 (08.06.1995 Gazette 1995/24)**

(54) **METHOD OF EXAMINING HORMONE-DEPENDENT TUMOR**

VERFAHREN ZUR UNTERSUCHUNG VON HORMONABHÄNGIGEN TUMOREN

PROCEDE POUR L'EXAMEN DE TUMEURS HORMONODEPENDANTES

(84) Designated Contracting States:
**AT BE CH DE DK ES FR GB IE IT LI LU NL SE**

(30) Priority: **03.12.1993 JP 33960893**

(43) Date of publication of application:
**22.11.1995 Bulletin 1995/47**

(73) Proprietor: **DAIICHI PHARMACEUTICAL CO., LTD.
Chuo-ku, Tokyo 103-8234 (JP)**

(72) Inventors:
• **NAKAMURA, Junji, Nishiura Haitsu 2-4
Shimotsugagun, Tochigi 329-05 (JP)**
• **YOSHIHAMA, Makoto
Utsunomiya-shi, Tochigi 321-01 (JP)**

(74) Representative: **Wakerley, Helen Rachael
Reddie & Grose, 16 Theobalds Road
London WC1X 8PL (GB)**

(56) References cited:
**EP-A- 0 250 262**

• **DATABASE WPI Section Ch, Week 9017 Derwent
Publications Ltd., London, GB; Class B01, AN
90-126933 XP002064005 & JP 02 067 296 A
(TAKEKAWA F) , 7 March 1990**
• **Journal of Japan Surgical Society, 90 (6),
TOSHIAKI UCHIMI: "Study on Local Production
of Estrogen, as Viewed from Activity of
Aromatase and Estrone Sulfatase in Breast
Cancer Tissues", p. 920-927.**
• **Br. J. Cancer, 60 (1), 1989, LONNING, P.E. et al.:
"Treatment of Breast Cancer with Aromatase
Inhibitors, Current Status and Future
Prospects", p. 5-8.**

EP 0 683 237 B1

**Description**

Technical Field

This invention relates to a method for examination of hormone-dependent tumors, more particularly to simultaneous judgment of the presence or absence of hormone-dependent proliferation of tumor tissue and the hormone synthetase. This invention provides guidelines for the diagnosis and medicinal treatment of tumors.

Background Art

There are a type of tumors called hormone-dependent tumors which generally occur in target organs of hormones and their growth can be promoted by those hormones, in other words, their growth are hormone-dependent. For example, mammary cancer depends on estrogen, prostate cancer depends on androgen and thyroid cancer depends on thyroid stimulating hormone (TSH). Hormonal endocrine therapies have been widely applied for the treatment of these hormone-dependent tumors.

Some types of mammary cancers are progressed by a female hormone, estrogen, and excision of estrogen-producing ovaries has been used for hormonal endocrine therapy. At present, administration of anti-estrogenic agent such as tamoxifen which binds to estrogen receptor in competition with estrogen is widely applied for the treatment of mammary cancer.

At a treatment of mammary cancers, an examination of the presence of estrogen receptor in the homogenate of tissue obtained by operation or biopsy from patients is generally used for identifying whether administration of anti-estrogenic agent can be applied or not. This test is based on the competitive binding of anti-estrogenic agent with estrogen to the estrogen receptor to exert the antitumor effect of the agent. The anti-estrogenic agent is applied after confirmation of the presence of estrogen receptor in the tumor. In practice, a correlation between the clinical effect of the anti-estrogenic agent and the presence of estrogen receptor is clinically significant. However, the estrogen receptor test is sometimes troublesome; because no estrogen-dependent proliferation can be expected without physiologically and functionally active estrogen receptor even if estrogen receptor is present. In fact, all tumors having estrogen receptor do not necessarily exhibit estrogen-dependent proliferation.

Recent studies show that some hormone-dependent diseases and tumors themselves produce hormones which are involved in the aggravation of diseases and proliferation of tumors. For example, some mammary cancers have an enzyme (aromatase) which synthesizes estrogen from androgen in the tumor tissues, and this synthesis of estrogen at high concentration in the tissue has been reported (Toshiaki Uchimi, J. Jpn. Surg. Soc., 90, 920-927, 1989).

A therapeutic treatment to suppress the synthesis of hormone in those diseases which produce the hormone in the tumorous tissue has been investigated. An enzyme inhibitor has been used to prevent local production of estrogen by an estrogen synthetase, aromatase, in a treatment of estrogen-productive mammary cancer.

The presence of hormone synthetase in the cancer tissue can be confirmed by direct determination of the enzymic activity in a homogenate of tissue obtained by operation or biopsy from the patients. Uchimi et al. (see above) reported a detailed procedure for the determination. Treatment with the aromatase inhibitor can be performed to tumor tissue after confirmation of both the presence of aromatase activity and estrogen-dependent proliferation. However, these troublesome tests of tumor tissue are rarely carried out, because of difficulty in sampling the target tissue and the limited amount of obtainable tissue for the determination of enzymic activity. Therefore, only an easily operable micro-analysis of estrogen receptor has been performed for the indication of the aromatase inhibitor. Unfortunately, these test methods can not confirm the presence of aromatase activity, and all tumors having estrogen receptor do not necessarily exhibit estrogen-dependent proliferation as mentioned above. Therefore, the test method may result in misdiagnosis. These problems can be found not only in estrogen-related diseases, but also in hormone-dependent diseases other than estrogen and tumors.

Tumors occurring in hormone-targeted organs can generally be categorized as shown in Table 1 by the presence or absence of hormone synthetase and hormone-dependent proliferation.

[Table 1]

| Type of tumor | Hormone synthetase | Hormone dependent proliferation |
|---|---|---|
| 1 | + | + |
| 2 | - | + |
| 3 | + | - |
| 4 | - | - |

The inventors have found a convenient method for the simultaneous determination of the presence or absence of hormone synthetase and hormone-dependent proliferation of tumors and accomplished the present invention.

Disclosure of Invention

An object of the present invention is to provide a simple and simultaneous examination method for the presence of hormone synthetase in the tissue of hormone-dependent tumors and for hormone-dependent proliferation of tumors.

Another object of the invention is to provide a method for the differentiation of a tumor type which has hormone synthetase and proliferates hormone-dependently (type 1 in the above Table 1) from the other tumor types.

The characteristic features of the present invention relate to an examination method for simultaneous judgment of hormone-dependent growth of tumor tissue and the presence of hormone synthetase. That is, tumor tissues are cultured in three different media - (a) a medium without additive, (b) a medium with added a hormone synthetase substrate, and (c) a medium with added a hormone synthetase substrate and an inhibitor of hormone synthetase. The growth of tumor cells in the three media is determined to give (i) the growth ratio of tumor cells in the medium with added the hormone synthetase substrate relative to that in the medium without the addition, and (ii) the growth ratio of tumor cells in the medium with added the hormone synthetase substrate and the hormone synthesis inhibitor to that in the medium without additive.

According to the present invention, tumors can be classified into hormone-dependent proliferation types 1 and 2, and non-dependent proliferation types 3 and 4 as shown in Table, 1, and the type 1 tumor can be easily differentiated from the other types of tumors.

The examination procedure of the present invention can be carried out by the following steps:

(1) Preparation of test sample by excision or biopsy of human tumor tissue.
(2) Culture of the sample in three different media - (a) a medium without additive, (b) a medium with added a hormone synthetase substrate, and (c) a medium with added a hormone synthetase substrate and an inhibitor of hormone synthetase.
(3) Determination of proliferation of the test sample (tumor tissue) in the three media.
(4) Confirmation of proliferation of tumors, and judgment.

This confirmation is performed by determinations of (i) the growth ratio of tumor cells in medium (b) compared to that in medium (a) and (ii) the growth ratio of tumor cells in medium (c) compared to that in medium (a). Thus hormone-dependent growth of tumor tissue and the presence of hormone synthetase can be simultaneously determined.

The examination procedure of the present invention will be explained using an estrogen-dependent tumor, a typical hormone-dependent tumor.

Mammary cancer can be illustrated as a representative estrogen-dependent tumor. In this case, estrogen is produced from androgen by an action of aromatase in the tumors and heretofore solely estrogen receptor has been examined.

Tumor tissues, for example, a tissue collected during operation or biopsy, is aseptically cut into small pieces and cultured. Any cultural medium used for cell culture can be applied and soft agar and collagen matrix can be illustrated, and Eagle or RPMI media is included as a cell culture medium. Any conventional tissue culture method can be used, as described in detail in publicly known literatures such as "Tissue Culture", ed. by Junnosuke Nakai et al. (Asakura Pub. Co., Ltd., 1976).

Such medium is added with testosterone, a substrate for estrogen synthetase aromatase, or a combination of testosterone and an aromatase inhibitor. The aromatase substrate testosterone is added to give a concentration of 1-1,000 nM, preferably 1-100 nM. Any compound which selectively inhibits aromatase activity without exhibiting cell toxicity can be used and 4-hydroxy-4-androsten-3,17-dione (generic name: formestane, Ciba-Geigy) and 14$\alpha$-hydroxy-4-androsten-3,6,17-trione (code name: NKS01, Snow Brand Milk Products Co., Ltd.) are enumerated.

The present invention will be explained using 14$\alpha$-hydroxy-4-androsten-3,6,17-trione (hereinafter abbreviated as NKS01). The concentration of aromatase inhibitor in the medium can be chosen according to its inhibitory activity and generally 0.01-100 $\mu$M of NKS01 is used. Tissue sections described above are placed in the prepared media and cultured by conventional methods for 1-14 days in general. Tissue proliferation is determined by the amount of labeled thymidine taken up into the tissue. Generally, determination of $^3$H-thymidine taken up in the tissue is used simply and conveniently.

When an amount of the growth is determined using an amount of $^3$H-thymidine taken up into the tissue, $^3$H-thymidine is added to the culture media 3-4 days prior to the end of culture, the tissue is collected at the end of culture, and $^3$H-thymidine in the collected tissue is measured with a liquid scintillation counter to estimate the growth of tissue. The determined amount of $^3$H-thymidine is divided by the nucleic acid content in the tissue to minimize the dispersion of the data between the tissues in each medium.

The growth rate is calculated from the relative rates of growth in the medium solely containing culture medium to those in the medium with added a substrate, and in the medium with added the substrate and an inhibitor. The growth rate is estimated by the following equation:

$$\text{Growth rate (\%)} = 100 \times [(\text{the amount of incorporated }^3\text{H-thymidine in the respective additive-containing medium/the concentration of DNA in the tissue of said medium})/(\text{the amount of incorporated }^3\text{H-thymidine in the additive-free medium/the concentration of DNA in the tissue cultured in said medium})].$$

The examination mentioned above for the suspected hormone-dependent tumor tissue in mammary cancer patients provides a new classification of lesions of patients with mammary cancer from a point of view not previously used. For example, if the excised mammary cancer tissue is estrogen-dependent and contains the estrogen synthetase, aromatase, then the tissue will grow in a medium with added testosterone, a substrate of aromatase, by estrogen converted from the testosterone. The promoted growth is suppressed by an aromatase inhibitor. Thus the excised tissue is classified as a type 1 hormone-dependent tumor in the above Table 1.

If the excised mammary cancer tissue sample lacks estrogen-dependent growth and/or estrogen synthetase, aromatase, then no stimulation of growth rate will be observed in the presence of testosterone.

Simultaneous and easy judgment of the presence or absence of hormonal synthetase and hormone-dependent proliferation in hormone-dependent tumors can be done by practice of the present invention. Furthermore, the results of the judgment are applicable to new guidelines for the classification of tumors and selection of medical treatment for the patients.

The type 1 tumor in the above Table 1 can be presumed to be treatable with an aromatase inhibitor and a suitable medical treatment course will be found for this type of the patients.

The best modes for carrying out the invention

The present invention is explained in detail by the following Examples and Reference example, but the scope of the present invention is not restricted by the Examples.

Example 1

An examination according to the present invention was performed on 21 patients diagnosed as having mammary cancer.

During operation on mammary cancer, a tissue section of 10 x 10 mm was excised from the center of tumor. The necrotic portion was aseptically removed immediately in Hanks' solution (Gibco Co., Ltd.) containing 100 U/ml each of penicillin and streptomycin, and then the tissue was cut to pieces of 0.5-1 x 0.5-1 mm with a scalpel. Four to five pieces of tissue were placed on collagen matrices in 24-wells microplate. The collagen matrices were prepared as follows; Spongy collagen matrix (Spongostan Co., Ltd.) was aseptically cut to pieces of one cm$^3$, placed each in a well of 24 well microplate, and a phenol red free modified Eagle MEM containing 10% fetal calf serum (FCS) and 100 U/ml each of penicillin and streptomycin (Gibco Co., Ltd.) was filled up to the surface of the matrix to prepare the medium.

The prepared 24 well microplate was placed in an atmosphere containing 5% $CO_2$ and cultured at 37°C for seven days. The steroid-free culture medium containing FCS was prepared of FCS treated with charcoal dextran and wholly replaced every other day.

The media used for tissue culture examination are composed of the following three groups:

(1) Control group (medium only),
(2) Substrate group (with 10 nM testosterone added),

(3) Substrate and enzyme inhibitor group (with 10 nM testosterone and one µM NKS01 added).

After seven days culture, the culture medium was changed to a medium containing 0.67 µ Ci of $^3$H-thymidine and cultured for another three days. After the culture, tissue tips on the collagen sponge in the well were transferred into tubes (each with added one ml of Hanks' solution containing 0.1 mg/ml of collagenase) and incubated at 37°C for eight hrs. The cell mass was dispersed by pipetting, then centrifuged at 3,000 rpm for 10 min. The formed precipitates were mixed with one ml of 100 mM Tris buffer, pH 7.5, containing 1% sodium dodecylsulfate and 200 µg/ml of proteinase and incubated at 50°C for three hrs.

The resultant solution was mixed with one ml of a mixture of phenol/chloroform/isoamyl alcohol (25:24:1) for a few min. The mixture was centrifuged at 3,000 rpm for 10 min. and the upper aqueous layer was taken out and mixed with 100 µl of 3M sodium acetate and 2.5 ml of cold ethyl alcohol. Precipitated DNA was gathered round a glass rod and soaked in 70%, 80% and 90% aqueous ethyl alcohol, successively, and dried in the air. The dried DNA was dissolved in one ml of 100 mM Tris buffer, pH 7.5, to give a DNA solution.

The concentration of DNA in the solution was determined by means of the absorbance at 260 nm and the $^3$H-thymidine content of the solution was measured with a liquid scintillation counter and expressed per one µg of DNA (dpm/µg DNA). The determination method of cell growth by $^3$H-thymidine incorporated per DNA is widely used and can be performed by conventional methods such as described by Fukuoka et al. (Masaaki Fukuoka et al., Acta Obst. Gynaec. Jpn., 43(12), 1667, 1973). The present example was performed according to the method cited above and the results are shown in Table 2.

[Table 2]

| Patient | Growth rate (%) | | Judgment |
|---|---|---|---|
| | Testosterone | Inhibitor | |
| A | 114.4 | 104.1 | |
| B | 96.9 | 95.0 | |
| C | 167.1 | 114.8 | |
| D | 100.7 | 97.7 | |
| E | 102.3 | 98.1 | |
| F | 114.6 | 98.6 | Type 1 |
| G | 104.0 | 107.4 | |
| H | 117.5 | 126.8 | |
| I | 120.5 | 99.8 | Type 1 |
| J | 101.2 | 117.7 | |
| K | 88.4 | 97.9 | |
| L | 120.9 | 97.2 | Type 1 |
| M | 129.7 | 77.4 | Type 1 |
| N | 100.3 | 95.2 | |
| O | 97.4 | 98.4 | |
| P | 156.4 | 101.3 | Type 1 |
| Q | 92.9 | 98.8 | |
| R | 108.8 | 95.2 | |
| S | 160.7 | 103.3 | Type 1 |
| T | 98.9 | 92.4 | |
| U | 92.6 | 93.6 | |

Mammary cancer tissues with estrogen-dependent proliferation and having aromatase were found in six out of 21 patients by the examination method of the present invention. The mammary cancers from these patients were hormone-dependent and presumed to be proliferated by the hormone synthesized in the tumor tissues.

Example 2

The applicability of aromatase inhibitor was examined according to the present invention for four human derived tumor cell lines. These cell lines had been tested by a conventional test method, confirmed the presence of an estrogen receptor and indicated for endocrine therapy, administration of aromatase inhibitor (Table 3).

**EP 0 683 237 B1**

[Table 3]

| Tumor cell line | Source | ER[1) |
|---|---|---|
| MCF-7 | Human mammary cancer | Yes |
| BG-1 | Human ovarian cancer | Yes |
| R-27 | Human mammary cancer | Yes |
| ISHIKAWA | Human endometrial cancer | Yes |
| ER[1): Estrogen receptor | | |

Each cell line was inoculated subcutaneously to axillary fossa of 7-week-old female nude mice at a rate of 1 x $10^5$ cells/0.1 ml/mouse to give solid tumors. The tumors grown to approximately one cm diameter were resected and tested by the method of the above mentioned present invention. The results are shown in Table 4.

[Table 4]

| Tumor cell line | Growth rate (%) | | Judgment |
|---|---|---|---|
| | Testosterone | Inhibitor | |
| MCF-7 | 635.3 | 102.0 | Type 1 |
| BG-1 | 288.7 | 117.6 | Type 1 |
| R-27 | 106.7 | 102.9 | Type 2 |
| ISHIKAWA | 307.0 | 279.2 | Type 2 |

These tumor cell lines were re-inoculated subcutaneously to axillary fossa of to 7-week-old female nude mice at a rate of 1 x $10^5$ cells/0.1 ml/mouse. The mice were divided into two groups each containing 10 mice when the tumor mass became approximately three mm diameter; one for control group without administration of an aromatase inhibitor and the other for test group with administration of an aromatase inhibitor everyday until the test was over. The aromatase inhibitors included 4-hydroxy-4-androsten-3,17-dione (general name: formestane, Ciba-Geigy), 4-(5,6,7,8-tetrahydroimidazo[1,5a]pyridin-5-yl)benzonitrile monohydrochloride (general name: fadorazole, Ciba-Geigy) and 14$\alpha$-hy-droxy-4-androsten-3,6,17-trione (code name NKS01, Snow Brand Milk Products Co., Ltd. disclosed in Japanese Laid-open (KOKAI) Patent Application No. 192794 (1988)).

The administration period of the inhibitors differs with the proliferation rate of the respective tumor in nude mice and was 28 days for BG-1, R-27 and ISHIKAWA, and 42 days for MCF-7. The longer and shorter axes of the tumors were determined on next day after the final administration with a measure such as vernier caliper. The volume of tumor was calculated by the following equation and the results are shown in Table 5.

Estimated volume of tumor =

$$\text{(longer axis)} \times \text{(shorter axis)}^2 \times 0.5$$

"Antitumor effect positive" was judged when there was a statistical significance in the volume of tumors between the control group and the inhibitor aromatase administered group. There are several statistical methods for the determination of the effects and T-test was used between the control group and the inhibitor aromatase administered group with a significance level of 5% or less. The antitumor effects of aromatase inhibitors against four tumor cell lines used in the present examination are cumulatively shown in Table 5.

[Table 5]

| Tumor cell line | Control group | Aromatase inhibitor | | |
|---|---|---|---|---|
| | | NKS01[1] | Formestane[2] | Fadorazole[3] |
| MCF-7 | 635±89[4] | 339±46 ($p < 0.05$)[5] Effective | 289±68 ($p < 0.05$) Effective | 255±41 ($p < 0.05$) Effective |
| BG-1 | 1,022±309 | 394±78 ($p < 0.05$) Effective | NT[6] | 401±86 ($p < 0.05$) Effective |
| R-27 | 1,298±266 | 1,145±245 Not effective | NT[6] | 1,321±564 Not effective |
| ISHIKAWA | 1,357±576 | 1,435±745 Not effective | NT[6] | NT[6] |

1): Oral administration 100 mg/kg/day

2): Subcutaneous administration 50 mg/kg/day

3): Oral administration 25 mg/kg/day (MCF-7) 60 mg/kg/day (BG-1) 3 mg/kg/day (R-27)

4): Estimated volume of tumor [Average±SD ($mm^3$)]

5): T-test result with those of the control group.

6): No experiment was performed.

The judged results for the treatment effect with the aromatase inhibitor by the examination of the present invention and the practical therapeutic effect of aromatase inhibitor were in good agreement as shown in the below mentioned Table 6. As shown by the above mentioned examples, the examination method of the present invention clearly differentiate indications of aromatase inhibitor therapy for patients with estrogen dependent tumor.

[Table 6]

| Tumor cell line | Indication by the present examination for aromatase inhibitor therapy | Type | Aromatase inhibitor | | |
|---|---|---|---|---|---|
| | | | NKS01 | Formestane | Fadorazole |
| MCF-7 | Indicated | 1 | Effective | Effective | Effective |
| BG-1 | Indicated | 1 | Effective | NT[1] | Effective |
| R-27 | Not indicated | 2 | Not effective | NT[1] | Not effective |
| ISHIKAWA | Not indicated | 2 | Not effective | NT[1] | NT[1] |

1): No experiment was performed.

## Claims

1. An examination method for the simultaneous determination of the presence of hormone-dependent proliferation of tumor tissue and the presence of hormone synthetase comprising of culturing samples of the tumor tissue in (a) a control medium, (b) the control medium with added substrate of the hormone synthetase, and (c) the control medium with added said substrate and inhibitor for said synthetase; ascertaining the relative growth ratios of tumor cells in media (i) (b) to (a) and (ii) (c) to (a); and simultaneously judging therefrom the hormone-dependent growth of tumor tissue and the presence of hormone synthetase.

2. The method according to claim 1 wherein the tumor tissue is obtained by transplantation and proliferation of human tumor tissue to nude mouse.

3. The examination method according to claim 1 or 2 wherein the hormone is estrogen.

4. The examination method according to claim 1 or 3 wherein the hormone synthetase substrate is testosterone and the hormone synthetase is aromatase.

**5.** The examination method according to claim 4 wherein the concentration of testosterone is 1-1,000 nM.

**6.** The examination method according to one of claims 1-5 wherein the hormone synthetase inhibitor is one or more inhibitors selected from the group composed of $14\alpha$-hydroxy-4-androsten-3,6,17-trione, 4-hydroxy-4-androsten-3,17-dione and 4-(5,6,7,8-tetrahydroimidazo[1,5-a]pyridin-5-yl)benzonitrile monohydrochloride.

**7.** A method for the determination of activity of an aromatase inhibitor for the treatment of tumours by examining the tumour according to any one of the preceding claims.

**Patentansprüche**

**1.** Untersuchungsverfahren zur simultanen Bestimmung der Anwesenheit einer hormonabhängigen Proliferation von Tumorgewebe und der Anwesenheit von Hormon-Synthetase, umfassend die folgenden Schritte: Kultivieren von Proben des Tumorgewebes in (a) einem Kontrollmedium, (b) dem Kontrollmedium mit zugegebenem Substrat der Hormon-Synthetase und (c) dem Kontrollmedium unter Zugabe des genannten Substrats und eines Inhibitors für die genannte Synthetase; Ermitteln der relativen Wachstumsverhältnisse von Tumorzellen in Medium (i) (b) bis (a) und (ii) (c) bis (a) und gleichzeitiges Beurteilen des hormonabhängigen Wachstums von Tumorgewebe und der Anwesenheit von Hormon-Synthetase anhand der Ergebnisse.

**2.** Verfahren nach Anspruch 1, wobei das Tumorgewebe durch Transplantation und Proliferation von humanem Tumorgewebe in nackter Maus erhalten wird.

**3.** Untersuchungsverfahren nach Anspruch 1 oder 2, wobei das Hormon Östrogen ist.

**4.** Untersuchungsverfahren nach Anspruch 1 oder 3, wobei das Hormon-Synthetase-Substrat Testosteron und die Hormon-Synthetase Aromatase ist.

**5.** Untersuchungsverfahren nach Anspruch 4, wobei die Konzentration von Testosteron 1 bis 1000 nM beträgt.

**6.** Untersuchungsverfahren nach einem der Ansprüche 1-5, wobei der Hormon-Synthetase-Inhibitor ein oder mehr Inhibitor(en) sind, ausgewählt aus der Gruppe bestehend aus $14\alpha$-Hydroxy-4-androsten-3,6,17-trion, 4-Hydroxy-4-androsten-3,17-dion und 4-(5,6,7,8-Tetrahydroimidazo[1,5-alpyridin-5-yl)benzonitril-monohydrochlorid.

**7.** Verfahren zum Bestimmen der Aktivität eines Aromatase-Inhibitors zur Behandlung von Tumoren durch eine Untersuchung des Tumors gemäß einem der vorherigen Ansprüche.

**Revendications**

**1.** Méthode d'examen pour déterminer simultanément la présence de prolifération hormono-dépendante de tissu tumoral et la présence d'hormone synthétase comprenant cultiver des échantillons de tissu tumoral dans (a) un milieu témoin, (b) le milieu témoin avec substrat ajouté de l'hormone synthétase, et (c) le milieu témoin avec ledit substrat et un inhibiteur de ladite synthétase ajoutés; constater les taux de croissance relatifs des cellules tumorales dans les milieux (i) (b) à (a) et (ii) (c) à (a); et juger simultanément à partir de ceux-ci la croissance hormono-dépendante du tissu tumoral et la présence d'hormone synthétase.

**2.** La méthode selon la revendication 1 dans laquelle le tissu tumoral est obtenu par greffe et prolifération de tissu tumoral humain chez la souris nue.

**3.** La méthode d'examen selon la revendication 1 ou 2 dans laquelle l'hormone est de l'oestrogène.

**4.** La méthode d'examen selon la revendication 1 ou 3 dans laquelle le substrat d'hormone synthétase est de la testostérone et l'hormone synthétase est de l'aromatase.

**5.** La méthode d'examen selon la revendication 4 dans laquelle la concentration de testostérone est de 1-1.000 nM.

**6.** La méthode d'examen selon l'une des revendications 1-5 dans laquelle l'inhibiteur d'hormone synthétase est un ou plusieurs inhibiteurs sélectionnés du groupe composé de 14 $\alpha$-hydroxy-4-androsten-3,6,17-trione, de 4-hydroxy-4-androsten-3,17-dione et de monochlorhydrate de 4-(5,6,7,8-tétrahydroimidazo[1,5-a]pyridin-5-yl)benzonitrile.

7. Méthode pour déterminer l'activité d'un inhibiteur de l'aromatase pour le traitement de tumeurs en examinant la tumeur selon l'une quelconque des revendications précédentes.